# EUROPEAN PATENT APPLICATION

(11) **EP 3 521 269 A1**
(43) Date of publication of application: **07.08.2019**
(21) Application number: 18155226.6
(22) Date of filing: 06.02.2018
(51) Int. Cl.: C07C 201/12, C07C 205/57

(54) **PROCESS FOR PREPARATION OF 1-NITROBENZENE-2-ALKYLOXYCARBONYL-5-CARBOXYLIC ACID**

(71) Applicant: Clariant Plastics & Coatings Ltd, 4132 Muttenz (CH)
(72) Inventor: TITTMANN, Rolf, 79400 Kandern (DE); HALDER, Somnath, Mumbai 400076, Maharashtra (IN); KUMTHEKAR, Sanjay, Pune 411038, Maharashtra (IN); GAYKAR, Dipak, Titwala (east), Thane 421605, Maharashtra (IN)
(74) Representative: Jacobi, Carola

(57) **Abstract**

The present invention relates to processes for preparation of 1-nitrobenzene-2-alkyloxycarbonyl-5-carboxylic acid. In particular, the present invention relates to preparation of 1-nitrobenzene-2-alkyloxycarbonyl-5-carboxylic acid by partial hydrolysis of diester of 1-nitrobenzene 2,5-dicarboxylic acid in presence of hydrogen chloride.

## Description

The present invention relates to processes for preparation of 1-nitrobenzene-2-alkyloxycarbonyl-5-carboxylic acid. In particular, the present invention relates to preparation of 1-nitrobenzene-2-alkyloxycarbonyl-5-carboxylic acid by partial acidic hydrolysis of diester of 1-nitrobenzene 2,5-dicarboxylic acid.

1-nitrobenzene-2-alkyloxycarbonyl-5-carboxylic acid is an important intermediate in the chemical industry used for the preparation of azo pigments. For example, Pigment Red 188 (PR188) is prepared from 1-nitrobenzene-2-methyloxycarbonyl-5-carboxylic acid. Recently, there has been reports on the usage of 1-nitrobenzene-2-alkyloxycarbonyl-5-carboxylic acid as an intermediate for preparation of certain pharmaceutical compositions as disclosed by WO-2012/059442, WO-2014/165126 and WO-2011/103321.

CS-138287 discloses a process for the preparation of 1-nitrobenzene-2-methoxycarbonyl-5-carboxylic acid (monoester) by the nitration of dimethyl terephthalate using sulfuric acid and nitric acid mixture. The nitro-intermediate formed is isolated followed by partial hydrolysis using large excess of 70 % aqueous sulfuric acid to obtain the monoester. The process has the disadvantage of generation of large amounts of highly acidic waste water. Moreover, it is impossible to prevent formation of explosive dimethyl ether from methyl alcohol resulting from the hydrolysis of the ester group.

DE-3001695 describes acidic ester hydrolysis of dialkyl ester of 1-nitrobenzene 2,5-dicarboxylic acid using aqueous nitric acid to avoid the formation of explosive dimethyl ether. This process has the disadvantage of formation of nitrous gases. Moreover nitric acid can react with methanol generated during hydrolysis and form methyl nitrate which is an explosive material. Further, nitric acid being a strong oxidizing agent may oxidize methanol to formaldehyde and to formic acid which is more toxic than methanol.

US-4507491 describes acidic ester hydrolysis of dialkyl ester of 1-nitrobenzene 2,5-dicarboxylic acid without isolation of nitro intermediate. The acidic ester hydrolysis is carried out in presence of sulfuric acid and a catalytic amount of an organic solvent which is completely or partially miscible with water and/or in presence of an emulsifier. The process has the disadvantages associated with using sulfuric acid and generation of large amounts of highly acidic waste water. Further, the formation of dimethyl ether cannot be discounted.

JP-06107599 discloses hydrolysis of dicarboxylic acid ester using alkali metal alcoholate in presence of an organic solvent. The organic solvent include aromatic hydrocarbons or halogenated hydrocarbons which are not environmentally benign.

Base hydrolysis of dialkyl ester of 1-nitrobenzene 2,5-dicarboxylic acid is known, and are disclosed by WO-2014/165126 A2, WO-2011/103321, US-5693636A, WO-2001/070737 A2 and Bioorganic and Medicinal Chemistry Letters Volume 24, pages 1742-1747. However, base hydrolysis is known for poor yields of 1-nitrobenzene-2-alkyloxycarbonyl-5-carboxylic acid. Moreover, they employ organic solvents like dioxane or tetrahydrofuran (THF).

There exists a need for a process for the preparation of 1-nitrobenzene-2-alkyloxycarbonyl-5-carboxylic acid (monoester) from a diester of 1-nitrobenzene 2,5-dicarboxylic acid (diester) that overcomes one or more of the shortcomings of the prior art methods.

The present invention provides a process for preparation of 1-nitrobenzene-2-alkyloxycarbonyl-5-carboxylic acid (monoester) of formula (I) by hydrolyzing a diester of 1-nitrobenzene 2,5-dicarboxylic acid (diester) of formula (II) in presence of hydrogen chloride (HCl). By the hydrolysis process, the ester group which is primarily at meta position to the nitro group is hydrolyzed while the ester group at the ortho position remains unaffected. 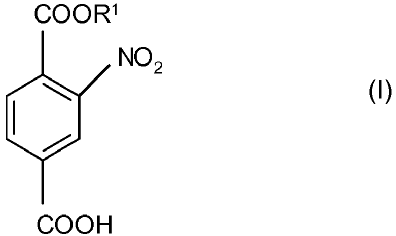 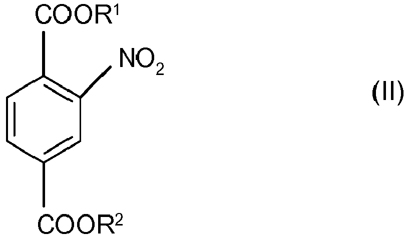 wherein
R¹ or R² are independently an alkyl group, alkenyl group or any combinations thereof. The alkyl group has preferably from 1 to 7 carbon atoms, more preferably from 1 to 4 carbon atoms and most preferably from 1 to 2 carbon atoms and can be linear or branched. Example alkyl groups include methyl, ethyl, n-propyl, iso-propyl, n-butyl, sec-butyl, iso-butyl and tert-butyl.

Suitable alkenyl group includes those having one or more double bonds and is preferably an alkenyl group having from 1 to 7 carbon atoms, more preferably from 1 to 4 carbon atoms and most preferably from 1 to 2 carbon atoms. Example alkenyl groups include 1-butenyl, 2-butenyl, 1-propenyl, allyl or vinyl. In a preferred embodiment, R¹ and R² are the same and is a methyl group.

The diester is provided in a reactor in an aqueous reaction medium. Optionally, a suitable solvent may be included in the reaction medium. Suitable solvents include any polar protic or aprotic solvents. Example such solvents include monoethylene glycol (MEG), diethylene glycol (DEG), glycerol and alcohols such as amyl alcohols or tertiary butanol.

The hydrogen chloride used in the present invention can be generated in-situ or can be provided as a gas, a liquid, or any combinations thereof. For example, a mixture of a carrier gas, such as nitrogen gas and hydrogen chloride of required partial pressure can be provided. Alternatively, hydrogen chloride can be provided in the reaction mixture as an aqueous solution, namely hydrochloric acid. The hydrochloric acid can be provided as a 20 % to 36 % solution, preferably as a 30 % to 36 % solution in the reaction mixture. In one embodiment, sodium chloride is provided in the reaction medium and sulfuric acid can be introduced to generate hydrogen chloride in-situ. Optionally, hydrogen chloride may be purified before use to remove any undesirable contaminants. A ratio of HCI to diester may vary depending on the concentration of HCI being used. Typically, HCI content may vary from 14 weight by weight (w/w) to 19 w/w times that of diester in the reaction medium.

The hydrolysis can be carried out at temperatures of 60 °C or above, more preferably at a temperature between 65 °C and 110 °C, most preferably at a temperature between 70 °C and 95 °C. The hydrolysis reaction can proceed for up to 10 hours, more preferably from 4 hours to 8 hours, and most preferably from 6 hours to 8 hours. It is also envisaged that the hydrolysis reaction can be carried out by subjecting the reaction mixture to a pulsed laser or continuous UV/visible light sources at a wavelength suitable for conducting the reaction. Such light sources include, for example Hanovia UV discharge lamps, sunlamps or even pulsed laser beams of appropriate wavelength or energy which are configured to irradiate the reaction mixture. In another embodiment, the hydrolysis reaction is conducted in presence of microwave energy. The microwave energy is a type of RF electromagnetic wave with frequency ranging from 300 GHz to 300 MHz. A typical microwave oven operates at 2.45 GHz and industrial scale ovens are typically about 915 MHz. For the purposes of the instant invention the microwave energy and frequency is selected for convenience with a preference for an optimum frequency wherein the frequency is matched to the dipole moment of the reactant. The heating rate of the molecules typically increases with dipole moment. As will be appreciated, reaction time of hydrolysis reaction conducted in presence of light or microwave source is faster compared to reaction time of traditional chemical reaction. The reaction time can vary from few seconds to several minutes.

The hydrolysis can be carried out as a batch process, semi-batch or a continuous process. In an exemplifying batch process, diester is charged to a reactor. Hydrogen chloride is provided as hydrochloric acid. Alternately, hydrogen chloride gas can be purged through the reactor. The reactor contents are heated to the desired temperature for the desired length of time. The reactor contents are then discharged from the reactor and either purified or sent to other equipment for further processing, or to storage.

In a semi-batch process, diester is fed to a reactor over a period of time throughout the reaction while hydrogen chloride is fed continuously throughout the reaction at the desired rate, which may be at constant flow, or constant pressure. After the reaction, the hydrogen chloride feed can be terminated and the reactor contents may be discharged for storage, purification or further processing.

In the large-scale production of chemicals it is often desirable to employ a continuous process since the economic advantage of doing so is usually greater than for batch processing. The continuous process may be, for example, a single-pass or a recycle process. In a single-pass process, diester pass through the process equipment once, and then the resulting effluent from the reactor is sent for purification or further processing. In such a scheme, hydrogen chloride is added as desired at a single point or at multiple points throughout the process equipment, which may include continuous stirred tank reactors, tubes, pipes or combinations thereof.

The hydrogen chloride is fed to the process equipment in any suitable manner. It is preferred that the process equipment is designed to ensure good dispersal of the hydrogen chloride throughout the reactor that is employed in the present process. Therefore, single or multiple spargers, baffles and efficient stirring mechanisms are desirable.

The equipment useful for the present invention may be any well-known equipment in the art. Suitable equipment may be fabricated of materials which are resistant to corrosion, and may include for example, metals, such as tantalum, suitable metallic alloys such as Hastalloy, or glass-lined equipment.

At the end of the hydrolysis reaction, the reaction medium is cooled down and diluted with cold water. 1-nitrobenzene-2-alkyloxycarbonyl-5-carboxylic acid can be separated from the reaction medium using any known separation techniques for isolating solids from liquids. In one method, reaction product is isolated using filtration. In another method, centrifugation is employed to separate the reaction product. The reaction product thus separated is washed with water and dried.

The process according to the invention excludes a high wastewater load and circumvents the formation of the dimethyl ether or methyl nitrate unlike prior methods using sulfuric acid and nitric acid. The formation of methyl chloride and dimethyl ether can be discounted in the present method as it requires drastic reaction conditions. A further advantage of the invention is the better yield of the 1-nitrobenzene-2-alkyloxycarbonyl-5-carboxylic acid compound and a better purity compared to prior methods. A purity of about 96% and a yield of above 90% have been achieved. Further, the process does not require a catalyst or a defoamer agent.

Without further elaboration, it is believed that one skilled in the art can, using the description herein, utilize the present invention to its fullest extent. The following examples are included to provide additional guidance to those skilled in the art in practicing the claimed invention. The examples provided are merely representative of the work that contributes to the teaching of the present application. Accordingly, the examples are not intended to limit the invention, as defined in the appended claims, in any manner.

### Examples

### Example 1

A mixture of 100 grams of 1-nitrobenzene-2,5-dicarboxylic acid dimethyl ester and 600-800 grams of concentrated hydrochloric acid was heated for 6 to 8 hours in a flask. The mixture was then quenched with cold water. The precipitate formed was filtered off with suction and washed with water. The yield of the 1-nitrobenzene-2-methyloxycarbonyl-5-carboxylic acid product was about 90%. The melting point was recorded and was found to be 174 - 176 °C for the product having a purity of 96 %.

## Claims

1. A process for preparing 1-nitrobenzene-2-alkyloxycarbonyl-5-carboxylic acid of formula (I) comprising, 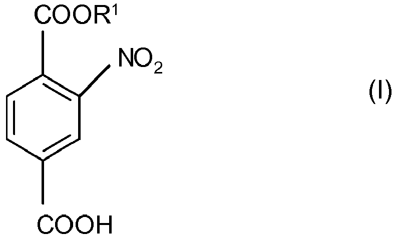 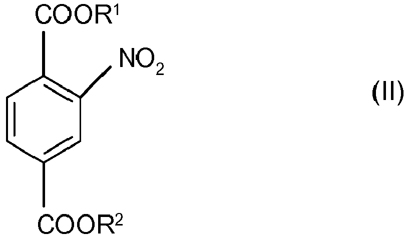 hydrolyzing a 1-nitrobenzene 2,5-dicarboxylic acid diester of formula (II) in presence of hydrogen chloride in an aqueous reaction medium.

2. The process of claim 1, wherein R¹ and R² are independently alkyl group, alkenyl group or any combinations thereof.

3. The process of claim 2, wherein the alkyl groups have 1 to 7 carbon atoms, and wherein the alkenyl groups have 1 to 7 carbon atoms.

4. The process of claim 2, wherein R¹ and R² of 1-nitrobenzene 2,5-dicarboxylic acid diester of formula (II) are the same and is a methyl group.

5. The process of claim 1, wherein the hydrogen chloride is generated in-situ or provided as a gas, a liquid or any combinations thereof.

6. The process of claim 1, wherein hydrolyzing the 1-nitrobenzene 2,5-dicarboxylic acid dialkyl ester is carried out at a temperature of from 60 °C to 110 °C.

7. The process of claim 1, wherein the reaction is conducted for a time period of up to 10 hours.

8. The process of claim 1, wherein the process is a batch, semi-batch or a continuous process.

9. The process of claim 1, wherein hydrolyzing the 1-nitrobenzene 2,5-dicarboxylic acid diester of formula (II) in presence of hydrogen chloride comprises hydrolyzing in presence of pulsed laser, continuous UV/visible light sources, microwave or any combinations thereof.

10. The process of claim 1 further comprising isolating the 1-nitrobenzene-2-alkyloxycarbonyl-5-carboxylic acid from the reaction medium.
